# EUROPEAN PATENT SPECIFICATION

(11) **EP 1 420 709 B1**
(45) Date of publication and mention of the grant of the patent: **08.08.2012**
(21) Application number: 02759455.5
(22) Date of filing: 26.08.2002
(51) Int. Cl.: A61B 17/70, A61F 2/44

(54) **UNILATERAL LAMINOPLASTY IMPLANTS**
UNILATERALE IMPLANTATE FÜR WIRBELSÄULENREKONSTRUKTION
IMPLANTS DE LAMINOPLASTIE UNILATERAUX

(30) Priority: 29.08.2001 US 942137
(43) Date of publication of application: 26.05.2004
(73) Proprietor: Synthes GmbH, 4436 Oberdorf (CH)
(72) Inventor: ANGELUCCI, Christopher, M., Schwenksville, PA 19473 (US); BOYER, Michael, L., II, Paoli, PA 19301 (US); PAUL, David, C., Phoenixville, PA 19460 (US); RYAN, Christopher, J., West Chester, Pennsylvania 19380 (US); SINHA, Amit, Drexel Hill, PA 19026 (US); WALTHER, Martin, West Chester, PA 19380 (US)
(74) Representative: Lusuardi, Werther
(86) International application number: PCT/US2002/027140
(87) International publication number: WO 2003/020142

(56) References cited:
- WO-A-00/44320
- WO-A-01/49220
- WO-A-99/21501
- WO-A-99/38461
- US-A- 6 001 130
- US-A- 6 156 037

## Description

The present invention relates to an implant according to the preamble of claim 1.

Various medical conditions may result in a reduction of the area within the vertebrae available for the spinal cord. Spinal stenosis is one such condition involving the narrowing of the canal in the center of the spine through which the spinal cord and nerve roots run. Spinal stenosis may result when the ligaments of the spine thicken and calcify (harden from deposits of calcium salts), or when bones and joints enlarge, and osteophytes (bone spurs) form. A herniated (bulging) disk may also place pressure on the spinal cord or nerve root. Furthermore, diseased bone or tumors may result in an ingrowth into the spinal cord area. This decreases the space (neural foramen) available for nerve roots leaving the spinal cord.

Two surgical methods currently exist to create additional room in the spinal canal. The first is called a laminectomy, and involves removal of the lamina (roof) of one or more vertebrae. A limitation of the laminectomy procedure is that it involves removal of the supporting structures at the back of the vertebrae which align the spinal column. The result may be that a patient suffers some postural deformity. To prevent such postural problems, a graft may be installed between the ends of the removed bone to span the void and reinstate the necessary support. The second procedure is called a laminoplasty, in which the targeted vertebra is cut, spread apart and a graft is inserted to permanently enlarge the space. Unlike the laminectomy, typically no bone material is excised during the laminoplasty procedure. Two different laminoplasty procedures are currently used. The first is called the unilateral or "open door" laminoplasty in which one side (lamina) of the vertebra is cut all the way through, while the other side is cut only half way to create a hinge. The vertebral element is then rotated about the hinge, and the graft is inserted into the opening, increasing the opening of the spinal canal. The second procedure is called the bilateral or "French door" laminoplasty in which the midline of the vertebra (spinous process) is cut all the way through, and the lamina are cut half way through, creating two hinges. The vertebral element is then opened at the bisected spinous process, and a graft inserted into the opening, again increasing the opening of the spinal canal.

Various materials may be used for the grafts installed during laminoplasty procedures. U.S. Patent Nos. 6,080,157 to Cathro et al. and U.S. Patent No. 5,980,572 to Kim et al. disclose the use of titanium, ceramic and nylon inserts. Further, using allografts taken from long bones such as the femur, humerus, tibia and fibula, for spinal fusion procedures is known, as disclosed by U.S. Patent No. 5,728,159 to Stroever et al. Allografts, as such bone grafts are called, are removed from a donor and processed using known techniques to preserve the allograft until implantation. Allografts have mechanical properties which are similar to the mechanical properties of vertebrae even after processing. The benefit of such property matching is that it prevents stress shielding that occurs with metallic implants. Allografts, unlike magnetic metals, are also compatible with magnetic resonance imaging (MRI) procedures, allowing more accurate ascertainment of fusion. Furthermore, allografts are naturally osteogenic providing excellent long term fusion with the patient's own bone.

Several different spacer designs have been used in laminoplasty procedures to the present. For example, the Cathro patent discloses a metal, nylon or teflon spacer for use in a unilateral laminoplasty procedure. The Cathro spacer is a rectangular plate having shouldered edges which engage the ends of the cut lamina, and is held in place by a spring mechanism. The difficulty with the Cathro spacer is that its operation relies on the continued satisfactory operation of the installed spring. Further, the Cathro device provides little available area for the packing of fusion enhancing (*i.e*. osteogenic) material. The Kim patent discloses a spacer for use in a bilateral laminoplasty procedure. The Kim spacer consists of inner and outer trapezoidal segments joined together by a rectangular segment. The tapered surface of the inner trapezoidal segment is designed to conform to the inner surface of the split spinous process halves, while the taper of the outer segment is designed to assume the shape of the removed spinous process tip. The Kim spacer seats on the resulting flat surface of bone. Like the Cathro device, the Kim device provides little area in which to pack osteogenic material to facilitate bone-implant fusion. Neither the CATHRO nor the KIM device use allograft as a spacer material, which may result in reduced propensity for fusion and the possibility for stress shielding.

From WO 99/38461 SYNTHES an intervertebral implant is known for fusing vertebrae. The two ends of the implant have channels for receiving an instrument. There is no disclosure with regard to a possible use for cut bone segments. The two ends are not designed as bone engaging portions and the channels are not adapted to engage and retain cut bone segments.

From WO99/21501 ZUCHERMAN an implant for spine distraction is known having cutouts which, however, are not positioned at the first and second ends of the hollow body of that implant but rather at the periphery of the hollow body. There is further no disclosure with regard to a possible use for cut bone segments.

The present invention provides implants for use in the spinal column, as claimed hereafter. Preferred embodiments of the invention are set forth in the dependent claims. The implants have a body portion having a length, a width and a depth and are configured to be insertable between first and second cut bone segments to maintain a desired distance between the bone segments as determined by a surgeon. The implants also have an outer surface and an inner surface defining a hollow portion, and first and second ends comprising bone engaging portions. In one embodiment, at least one of the bone engaging portions has a cutout configured and adapted to retain at least one of the cut bone ends. The perimeter of the outer surface of the implant may comprise a substantially geometric shape such as an ellipse or a circle.

The implants may be formed of biocompatable metal (e.g. titanium, stainless steel, etc.) or polymer, or of bone allograft material. For the implants formed of bone allograft material at least one of the implant's bone engaging portions may be comprised of partially, substantially, or fully demineralized bone.

The implants formed of bone allograft material may also have bone flaps connecting to and extending from the implant body portion at one or both ends. Each bone flap may be comprised of partially, substantially, or fully demineralized bone, or it may have a notch in the region where the bone flap connects to the implant body. Each bone flap may have at least one hole suitable for receiving a bone fastener for securing the implant to the bone segments.

The implants formed of bone allograft will further have an inner surface that may be defined by the intermedullary canal of the donor bone, or it may be configured so that the volume of the hollow portion is substantially greater than the intermedullary canal of the donor bone.

Where an implant is provided with both bone engaging portions having cutouts, the cutouts can have a substantially concave arcuate shape. Where a bone engaging portion comprising a cutout is provided, the at least one cutout may have at least two angled faces.

The implants further may be provided with at least one hole suitable for attaching a suture to secure the implant to the adjacent bone upon installation.

The implants may also be provided with at least one bone engaging portion having surface projections to reduce the possibility for slippage between the bone engaging portion and the respective cut bone end. These bone engaging portions may be saw tooth grooves, or they may comprise individual pyramidal teeth.

For implants formed of a bone allograft material, one or bothbone engagement portions may be comprised partially, of substantially, or fully demineralized bone. One or both bone engaging portions may also have surface projections to reduce the possibility of slippage between the bone engaging portion and the respective cut bone end.

In a different embodiment, an implant for use in the spinal column also may be provided having first and second plates connected by an intermediate portion whose thickness is smaller than the height of the first and second plates. The first and second plates may have bone engaging portions for engaging the first and second bone segments produced during a laminoplasty procedure. The bone engaging portions further may comprise concave or arcuate surfaces configured to retain the first and second bone segments. The bone engaging portions also may be angled with respect to each other. The implant may be provided wherein the first and second plates and the intermediate portion form a substantially U-Shaped implant. The implant of this design may be made of a biocompatable metal (e.g. titanium, stainless steel, etc.) or polymer, or from a cortical bone allograft. Where the implant is made of allograft material, one or both of the bone engaging portions may be comprised of partially, substantially, or fully demineralized bone.

A method not forming part of the invention for providing a desired distance between first and second cut bone ends of the spine comprises the steps of: cutting a vertebra to produce first and second cut bone ends; separating the bone ends to make a space therebetween; providing a plate having a length greater than the space between the bone ends, the plate having bone engaging portions at either end, where each bone engaging portion is able to receive at least one fastener, and further where the plate is permanently deformable to allow the surgeon to conform the bone engaging portions to correspond with the cut bone ends; engaging the bone engaging portions with the bone ends; providing at least two bone fasteners and inserting them into the fastener receiving portions, and engaging the fasteners with the respective bone ends. The method may further comprising the additional intermediate step of deforming the plate to conform it to the adjacent cut bone ends. The method may further comprise the step of providing a plate having a plurality of bone fastener holes.

A further method not forming part of the invention for providing a desired space in the spinal canal comprises the steps of: cutting one lamina of a vertebra all the way through to produce first and second cut bone ends; cutting the other lamina of the same vertebra partially to create a hinge; providing an implant having a body portion and a longitudinal axis and first and second ends, where the first and second ends have bone engaging portions and at least one of the bone engaging portions comprises an arcuate cutout, the cutout having a centerline running parallel to the longitudinal axis of the implant, and the cutout centerline being offset from the longitudinal axis; separating the bone ends far enough to accept the implant; placing the implant between the bone ends, and contacting at least a part of the bone ends with the implant bone engaging portions. The method may iniclude the additional steps of: providing a plate having first and second ends comprising bone engaging portions, which portions further comprise at least one bone screw receiving portion, where the length of the plate is greater than the space created between the bone ends so that the bone engaging portions can engage the bone ends; placing the plate over the installed implant so that the plate covers at least part of the implant and the bone screw receiving portions contact the first and second bone ends; inserting at least one bone screw within each bone screw receiving portion of the plate, and engaging at least one bone screw with the surface of each bone end.

An further method not forming part of the invention for providing a desired space in the spinal canal comprises the steps of: cutting one lamina of a vertebra all the way through to produce first and second cut bone ends; cutting the other lamina of the same vertebra partially to create a hinge, providing an implant made of bone allograft material having a body portion and first and second ends, where the first and second ends have bone engaging portions and at least one of the bone engaging portions is comprised of partially, substantially, or fully demineralized bone; separating the bone ends far enough to accept the implant; placing the implant: between the bone ends, and contacting at least a part of the bone ends with the implant bone engaging portions. The method may also include the additional steps: of providing a plate having first and second ends comprising bone engaging portions, which portions comprise at least one bone screw receiving portion, where the length of the plate is greater than the space created by the bone ends so that the bone engaging portions can engage the bone ends; placing the plate over the installed implant so that the plate covers at least part of the implant and the bone screw receiving portions contact the first and second bone ends; inserting at least one bone screw within each bone screw receiving portion of the plate; and engaging at least one bone screw with the surface of each bone end.

### BRIEF DESCRIPTION OF THE DRAWINGS

The features and advantages of the implant according to the invention will become more readily apparent from the following detailed description of the invention in which like elements are labeled similarly and in which:
FIGS. 1A, 1B and 1C are perspective, end and top views of the first embodiment of the implant, for use in a unilateral laminoplasty procedure;
FIGS. 2A and 2B are side and top views of the implant of FIG. 1 installed between the cut lamina segments of a vertebra during a unilateral laminoplasty procedure;
FIGS. 3A and 3B are a perspective view of a retaining plate of the present invention, and a side view of two such retaining plates installed over the implants of FIGS. 2A and 2B;
FIGS. 4A and 4B are perspective and side views of a second embodiment of the implant, a unilateral implant incorporating demineralized bone flaps;
FIGS. 5A, 5B and 5C are perspective, side and end views of a third embodiment of the implant, for use in a bilateral laminoplasty procedure;
FIGS. 6A and 6B are side and section views of the implant of FIG. 5 showing the incorporation of a channel to accept the corresponding arms of a set of distractor pliers used to install the implant;
FIG. 7 is a detail view of the end of the implant of FIG. 5B showing a preferred embodiment of the surface projections used to facilitate retention of the implant between cut spinous process segments.
FIGS. 8A, 8B and 8C are perspective, end and side views of a fourth embodiment of the implant, for use in a bilateral laminoplasty procedure;
FIG. 9A and 9B are front and top views of the implants of FIGS. 7 and 8 installed between the cut spinous process segments of a vertebra during a bilateral laminoplasty procedure;
FIGS. 10A, 10B and 10C are perspective, end and top views of a fifth embodiment of the implant, for use in a unilateral laminoplasty procedure;
FIGS. 11A, 11B and 11C are top, side and end views of a sixth embodiment of the implant, for use in a unilateral laminoplasty procedure; and
FIGS. 12A and 12B are perspective views of seventh and eighth embodiments of the implant, for use in unilateral laminoplasty procedures.

### DETAILED DESCRIPTION OF THE PREFERRED EMBODIMENTS

Preferred embodiments, features and aspects of an implant adapted to be used in unilateral and bilateral laminoplasty procedures are described, in which a portion of a targeted vertebra is cut, the space available for the spinal cord and associated nerves is expanded, and an implant is installed between the cut segments of bone.

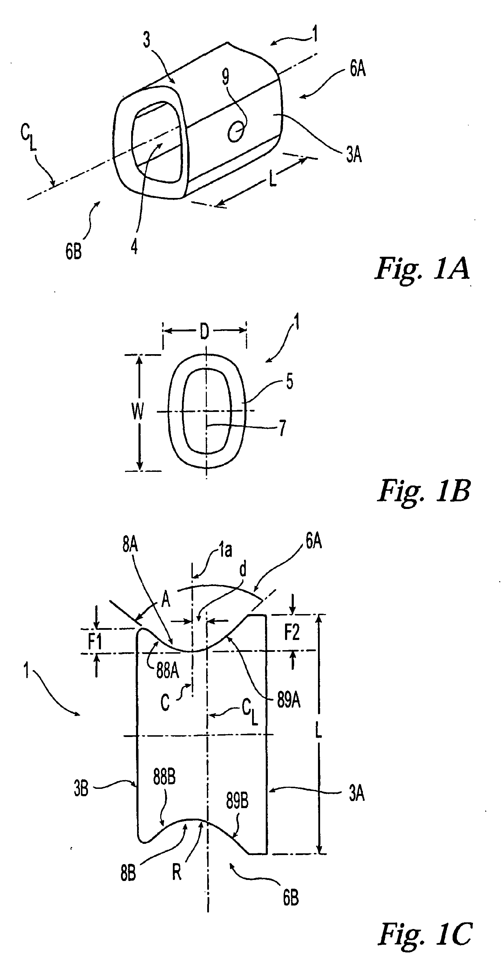

Referring more particularly to the drawings, FIGS. 1A, 1B and 1C show an implant for use in a unilateral or "open door" laminoplasty. The implant 1 has a longitudinal axis "CL," a length "L," a wall 5 defining an outside surface 3 and an inside surface 4, and first and second ends 6A, 6B. Inside surface 4 communicates with first and second ends 6A, 6B to define a hollow central region 7 of the implant. Outside surface 3 has an outer side region 3A and an inner side region 3B such that when the implant is installed between cut segments of lamina, outer side region 3A faces outward away from the spinal canal, while inner side region 3B faces inward toward the spinal canal. The implant 1 further has a depth "D" which is the distance between outer side region 3A and inner side region 3B. Implant 1 also has a width "W" which is the distance between opposing outer surfaces 3 measured along a line drawn perpendicular to a line defining the depth "D." Length "L" preferably should be between about 11.5 millimeters (mm) to about 15.5 mm; depth "D" preferably should be between about 5.5 mm to about 6.5 mm; and width "W" preferably should be between about 8.0 mm to about 9.5 mm.

The shape and size of outside surface 3 is not critical and, therefore, any implant configuration can be used preferably so long as the first and second ends 6A, 6B provide sufficient contact area with the lamina ends, and the implant 1 does not interfere with other anatomy, and does not intrude on the spinal cord space. In a preferred embodiment, however, the outside surface 3 is configured such that the shape of the implant, when viewed from the end, displays the form of a substantially geometric shape (e.g. ellipse, oval, circle, etc.). In this embodiment the exterior dimensions of the implant also approximate those of the outside surface of the cut lamina segments between which the implant is installed. Although implants having cross sections of greater or lesser proportion than the lamina to which they attach will function properly, for aesthetic purposes and in an attempt to minimize the amount of material introduced into a patient's body, the outer surface of the implant should preferably not extend beyond the outer surface or the adjoining bone.

In a further embodiment, the inside surface 4 of the implant 1 may be machined so that the hollow central region 7 approximates the configuration and geometry of the implant exterior (*i.e*. form an ellipse or oval shape). The hollow central region may be designed to be packed with osteogenic material such as bone chips, etc. to facilitate fusion of the implant with the patient's lamina. Preferably, the central region may be as large as possible to enhance fusion of the implant to the patient's lamina. The thickness of wall 45 preferably should be between about 1.00 to about 1.50 mm; more preferably about 1.25 mm. Preferably the thickness of wall 5 should not be less than about 1.0 mm to ensure the implant retains sufficient strength to withstand the stresses imparted on the spine.

The implant 1 may be fabricated from a biocompatable metal (*e.g*. stainless steel, or titanium, etc.) or polymer, or from allograft material preferably taken from a long bone (*e.g*. femur, tibia, fibula, humerus). Where the implant is an allograft, the inside surface 4 and hollow central region 7 may be defined by the intermedullary canal of the donor bone. The hollow center may be left as such, or the inner surface 4 may be machined, as with other implant materials, to maximize the space available for packing with osteogenic material. Again, the thickness of the implant wall 5, preferably is not reduced to less than about 1.00 mm.

During the unilateral laminoplasty procedure, the targeted lamina is cut in half and the segment attached to the spinous process is rotated or swung out to increase the area available for the spinal cord and associated nerves. Subsequent to this rotation, the lamina segments no longer reside along the same axis, but instead the ends are disposed at an angle with respect to each other. Implant 1 is substantially straight along its length, and so to accommodate this angular displacement of the lamina, first and second ends 6A, 6B incorporate arcuate cutouts 8A, 8B to grasp and retain the cut lamina segments. Viewed from the top of the implant (FIG. 1C), these arcuate cutouts 8A, 8B are generally concave and may be circular in shape, or they may consist of a cutout spanning an obtuse angle and converging to a small radius at the crotch of the first and second ends 6A, 6B. Arcuate cutouts 8A, 8B have a centerline 1a which runs parallel to the longitudinal axis of the implant 1. The centerline 1a of the arcuate at least one of the cutouts is offset with respect to that axis to further improve retention of the cut and displaced lamina ends. In a further embodiment, the centerlines 1a of the arcuate cutouts may each be offset on an opposite side of the implant centerline to facilitate retention of the implant in cases where the angle between the cut and spread lamina is more severe, such as when the surgeon spreads the lamina segments as wide as possible to provide maximum additional space for the spinal cord and associated nerves.

In the preferred embodiment, shown in FIG. 1C, each arcuate cutout 8A, 8B comprises first angled faces 88A, 89A and second angled faces 88B, 89B, respectively, which meet at crotch "C" to form a face angle "A." Preferably, face angle A is about 100 degrees. Crotch radius "R," comprises the transition between the first and second angled faces. Crotch radius "R" is preferably about 2 mm. Each arcuate cutout further comprises first and second face depths "F1" and "F2." The first and second face depths are a measure of the depth of the crotch relative to the inner side region 3B and outer side region 3A of the implant, and will be different lengths whenever the centerline 1a of the arcuate cutout is offset from the centerline "CL" of the implant 1. Preferably first face depth "F1" is about 1.25 mm, and second face depth "F2" is about 1.5 mm. Each arcuate cutout 8A, 8B also has a centerline offset "d," which is the degree to which the arcuate cutout 8A, 8B is shifted from the centerline "CL" of the implant 1. Preferably, the centerline offset "d" is from about 0 to 2.5 mm toward the inner side region 3B of implant 1. The face depth "F1" of the first and 6A of the implant 1 may be the same or different than the face depth "F1" of the second end 6B. Likewise, the face depth "F2" of the first end 6A may be the same or different than the face depth "F1" of the second end 6A.

In a further embodiment of the implant comprising allograft material, first and second ends 8A, 8B may comprise regions of partially, substantially, or fully demineralized cortical bone to further facilitate fusion of the implant to the lamina. Preferably the demineralized bone portion comprises the entire surface of each first and second end 6A, 6B of the implant 1. Preferably, the depth of the demineralized portion will be up to about 2 mm.

The implants further may incorporate at least one suture hole 9 in the implant wall 5 to allow the surgeon the option of suturing the implant to the cut lamina ends. These suture holes 9 may vary in number and size, with the only limitation being that they should not be so large or numerous as to compromise the strength or integrity of the implant.

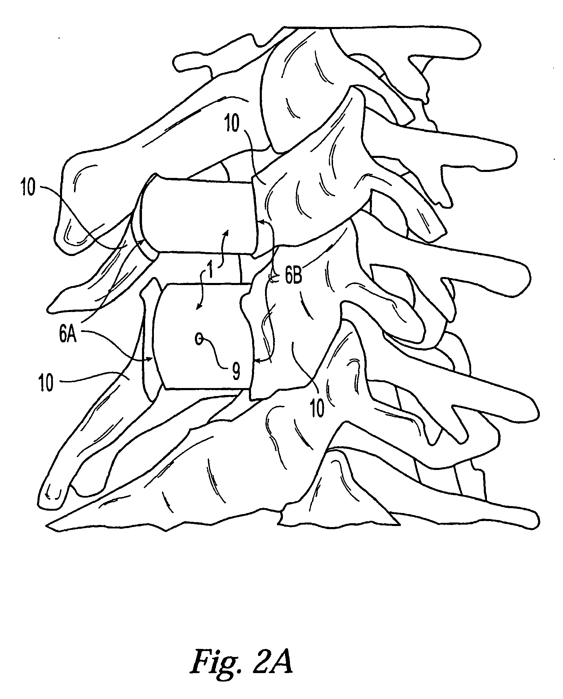

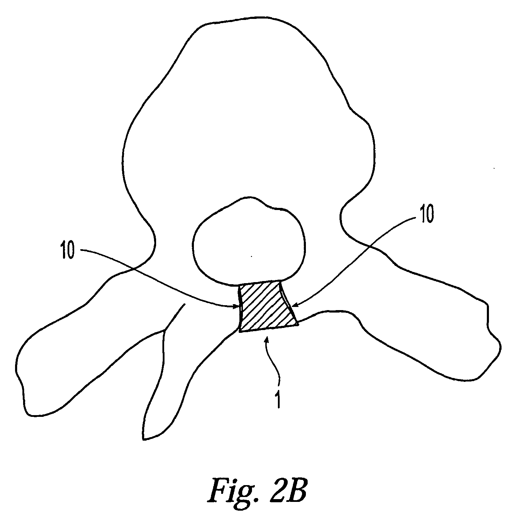

FIGS. 2A and 2B are side and top views of the implant of FIG. 1 installed in a patient between the cut lamina ends in a unilateral laminoplasty procedure. In FIG. 2A two different sized implants 1 are installed on the cut lamina segments 10 of adjacent vertebrae, to illustrate application of the implant design to bones of different size. FIG. 2B shows the interaction between the implant and the cut vertebra segments 10.

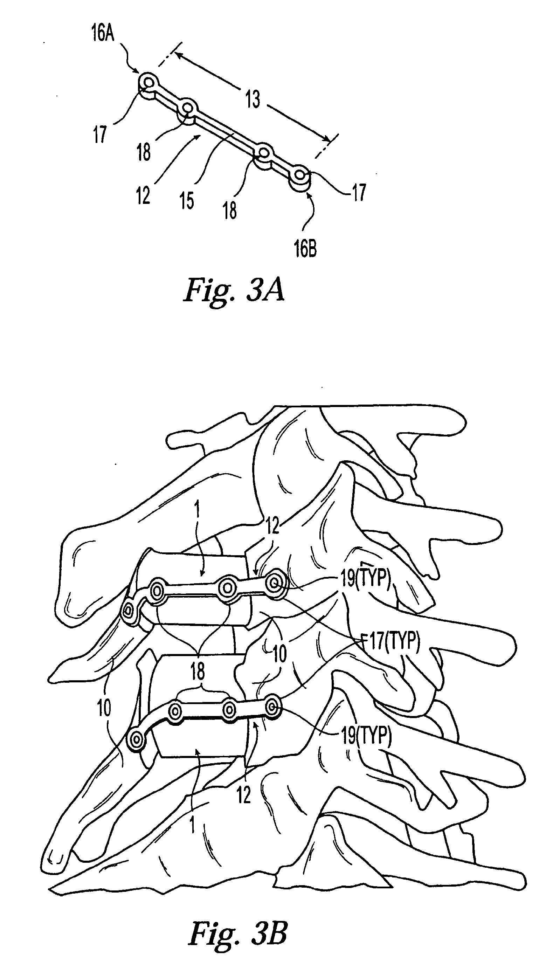

The design of the bone engaging ends 6A, 6B of the implants 1 are sufficient to ensure retention of the implants 1 between the cut ends of lamina 10. Some surgeons, however, desire an additional measure of assurance that the implants 1 will not loosen or otherwise be expelled from between the lamina ends 10. The implant, therefore, provides for the optional installation of a plate 12 to be secured over an installed implant in a unilateral laminoplasty procedure. FIG. 3A is a perspective view of a plate 12 which may be installed to secure the implant 1 of FIGS. 1 & 2, to ensure the implant 1 is not expelled from the cut lamina ends 10. Plate 12 has a length 13, a thickness 14 and a body portion 15 with first and second ends 16A, 16B comprising bone engaging portions 17 and implant engaging portions 18. As shown in FIG. 3A the bone engaging portions 17 and implant engaging portions 18 may consist of the holes adapted for receiving bone screws 19 or hooks 20 (not shown) capable of grasping bone screws installed in the lamina and/or implant. Each side of plate 12 may have one or more bone engaging portions 19 and one or more implant engaging portions 18. In a further embodiment the plate 12 may be flexible to allow the surgeon to form it to the individual contour of the patient's spine, thereby achieving a tight fit between components. The plates may be fabricated from a biocompatable metal or other material known in the art that would be suitable for long term retention of an implant 1.

Instead of a single plate 12, smaller plates without connecting body portion 15 may be utilized, each plate comprising at least one bone engaging portion 17 and one implant engaging portion 18.

FIG. 3B is a side view of the implants 1 installed in FIG. 2A, further showing the installation of optional plates 12 of FIG. 3A. Bone screws 19 are installed to secure the plates 12 to both the respective opposing lamina segment 10, and the implant. In this embodiment, bone screws are also installed in the screw holes 18 of the implant engaging portion, to secure the plates to the implants 1. Also in this embodiment, the plates are flexible and are bent to assume the varying contour of the lamina segments and the implant. More than one optional plate may be used to secure the implant to the lamina.

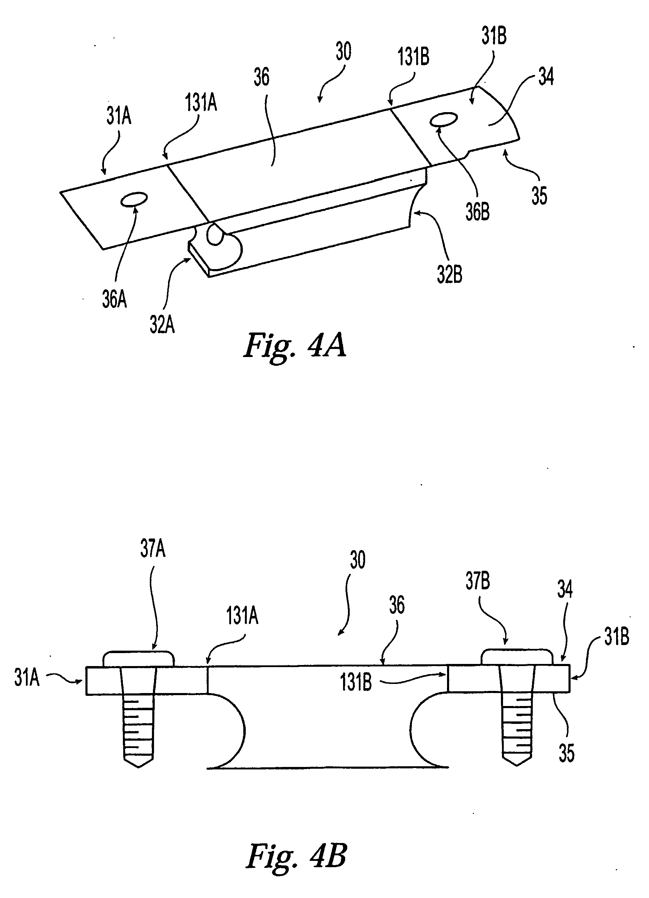

FIGS. 4A and 4B show perspective and side views of an allograft implant 30 which incorporates the design features of the implants of FIG. 1, but which further includes a pair of bone flaps 31A, 31B disposed at first and second ends 32A, 32B of the implant 30. These bone flaps are used to secure the implant 30 to the respective cut ends of lamina in a unilateral laminoplasty procedure. At least a portion of each flap comprises demineralized bone. Demineralization of the flaps, but not the implant, provides the implant with flexible attachment points which may be contoured to conform to the shape of the adjacent lamina. Bone flaps 31A, 31B comprise thin, flat, rectangular segments of allograft having an outer surface 34 and a bone engaging surface 35. The outer surfaces 34 of the flaps preferably are the same width as, are contiguous with, and extend axially like wings from the outer surface 36 of the implant 30. In a preferred embodiment, bone flaps 31A, 31B are machined from the same segment of donor bone as implant 30. At least a portion of flaps 31A, 31B may be demineralized using any commercially acceptable process (*e.g*. hydrochloric acid bath, etc.) that will render the resulting flaps flexible. Flaps 31A, B are provided with holes 36A, 36B suitable for receiving bone screws 37A, 37B which are used to secure the bone flaps 31A, 31B and implant 30 to the adjacent cut lamina ends.

In another embodiment, these bone flaps may not be demineralized, but instead each bone flap may comprise a notch 131A, 131B in the respective region where the bone flaps 31A, 31B connect to the implant 30. Notches 131A, 131B may be any type of notch or reduction in the thickness of the bone flap appropriate to provide flexibility for placing the flaps on the adjacent laminae surfaces, while retaining the requisite strength to ensure the bone flaps will not separate from the implant during installation.

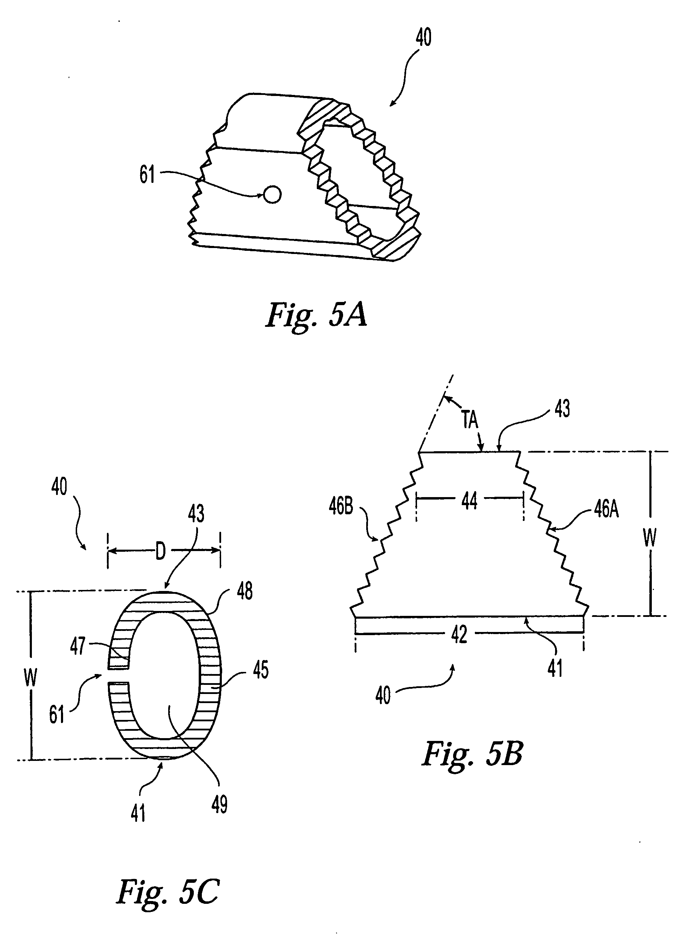

FIGS. 5A, 5B and 5C show an embodiment of an implant for use in a bilateral or "french door" laminoplasty procedure, in which the spinous process of a targeted vertebra is bisected along the sagittal plane and the segments separated to enlarge the spinal canal. The implant 40 has a wall 45 having an inside surface 47 and an outside surface 48, and first and second ends 46A, 46B. The outside surface 48 has an outer side region 41 having an outer side length 42 and an inner side region 43 having an inner side length 44. Inside surface 47 communicates with first and second ends 46A & 46B to define a hollow central region 49 of the implant. The implant 40 has a generally trapezoidal shape when viewed from the side (FIG. 5B), and inner side region 43 forms angle "TA" with respect to the first and second ends 46A, 46B. This trapezoidal configuration allows the implant first and second ends 46A, 46B to conform to the cut, angled surfaces of the spinous process segments to which the implant will eventually fuse. Inner side length 44 preferably is from between about 6.0 mm to about 10 mm, and angle "TA" preferably is from between about 50 to about 70 degrees.

The shape and size of outside surface 48 is not critical and, therefore, any implant external configuration can be used preferably so long as first and second ends 46A, 46B provide sufficient contact area with the cut spinous process segments, does not project out from between the bone segments so far as to interfere with other anatomy, and does not intrude on the spinal cord space For aesthetic purposes and in an attempt to minimize the amount of new material introduced into a patient, however, the outside surface 41 of the implant 40 should preferably not extend beyond the outside surface of the cut spinous process segments. In a preferred embodiment the outside surface 41 of the implant 40 is configured such that the outside surface 41, when viewed from the end, displays the form of a substantially geometric shape (e.g. ellipse, oval, circle, etc.) (FIG. 5C).

In a further embodiment, the inside surface 43 of the implant 40 may be machined so that the hollow central region 49 approximates the configuration and geometry of the implant outside surface 41 (*i.e*. an ellipse or oval). The hollow central area is designed to be packed with osteogenic material such as bone chips, etc. to facilitate fusion of the implant with the patient's cut spinous process segments. Preferably, this center area may be made as large as possible to facilitate the fusion process.

The thickness of wall 45 preferably should be from between about 1.00 to about 1.50 mm; more preferably about 1.25 mm. Preferably the thickness of wall 45 should not be less than about 1.0 mm to ensure the implant retains sufficient strength to withstand the stresses imparted on the spine associated with daily living.

The implant 40 may be fabricated from a biocompatable metal (*e.g*. stainless steel, or titanium, etc.) or polymer, or from allograft material preferably taken from a long bone (*e.g*. femur, tibia, fibula, humerus). Where the implant is fabricated from metal or polymer, it may be provided in a solid form. Preferably, however, the implant should incorporate a hollow region, and the inside surface 44, should be formed to maximize the space available for packing with osteogenic material while maintaining adequate wall thickness. Where the implant is an allograft, the inside surface 44 and hollow center 49 may be defined by the intermedullary canal of the donor bone. The allograft may be left in this state, and the hollow central region 49 packed with osteogenic material. Preferably, however, the inside surface 44 of the allograft will be machined and the hollow central region 49 enlarged to maximize the space available for packing with osteogenic material.

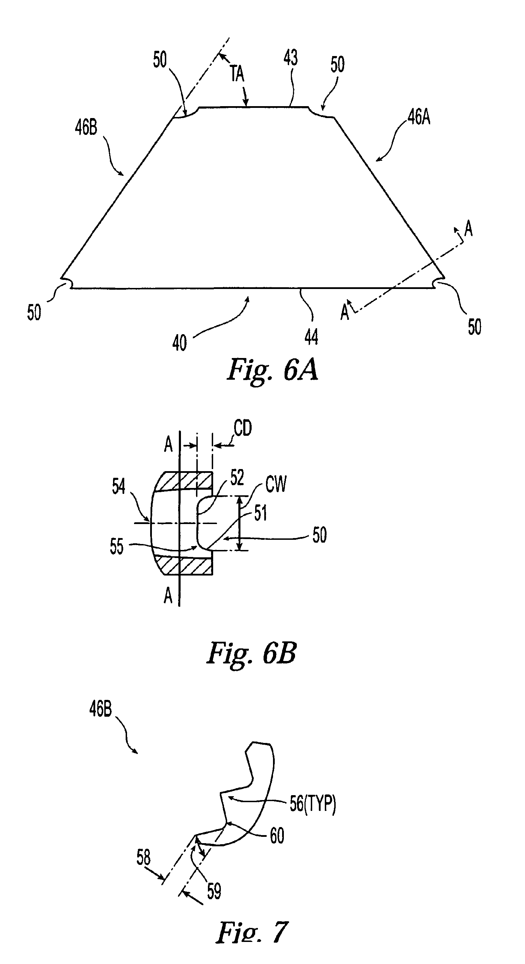

FIGS. 6A and 6B show first and second ends 46A, 46B of implant 40 each incorporating a channel 50 to accept the corresponding arms of a set of distractor pliers (not shown) which may be used to separate the bisected spinous process segments during the bilateral laminoplasty procedure. Each channel 50 has two sidewalls 51 each having a depth "CD", a bottom surface 52 having a width "CW" and a centerline 54 which is formed by a line extending along the implant 40 from inner side region 43 to outer side surface 41. Preferably, each channel 50 may incorporate a radiused transition 55 between the sidewalls 51 and the bottom surface 52. In a further preferred embodiment, the channel runs from the inner side region 43 to the outer side surface 41 of each end 46A, 46B of the implant. The specific dimensions of the channels is not critical, but should be configured to accept the distractor arms used during the distraction and insertion portion of the procedure. Preferably, the channel bottom surface width "CW" is about 4 mm, and the sidewall depth "CD" is about 1 mm.

FIG. 7 shows a further embodiment of bilateral laminoplasty implant 40, in which first and second ends 46A, 46B comprise surface projections to improve pre-fusion retention of the implant 40 between respective cut spinous process segments. In a preferred embodiment, a plurality of saw-tooth serrations 56 having a height 58 and a tooth angle 59 are provided. Preferably the serrations are oriented to run vertically when the implant 40 is installed in the patient. Height 58 and tooth angle 59 are defined with respect to the respective planes formed by implant first and second ends 46A, 46B. Height 58 is measured from the trough 60 of each serration, while tooth angle is measured from the plane formed by the implant first and second ends 46A, 46B. Preferably, height 58 is about 0.5 mm, tooth angle 59 is about 45 degrees, and the distance between troughs 60 is about 1.2 mm. While these dimensions and profile are preferred, other suitable surface profiles (*e.g*. pyramidal teeth, etc.) may be used to ensure implant retention.

In a further embodiment of the implant 40 comprising allograft material, first and second ends 46A, 46B may comprise regions of partially, substantially, or fully demineralized cortical bone to further facilitate fusion of the implant to the lamina. Preferably the demineralized bone portion may comprise the entire surface of each first and second ends 46A, 46B of the implant 40. Preferably the depth of the demineralized portion of will be up to about 2 mm.

The implant 40 may also incorporate a plurality of sutures holes 61 (see FIG. 5C) formed through the implant wall 45 to allow the surgeon to secure the implant to the cut spinous process segments. These suture holes 61 may vary in number, size and position, with the only limitation being that their size, position and number preferably should not compromise the strength and integrity of the implant.

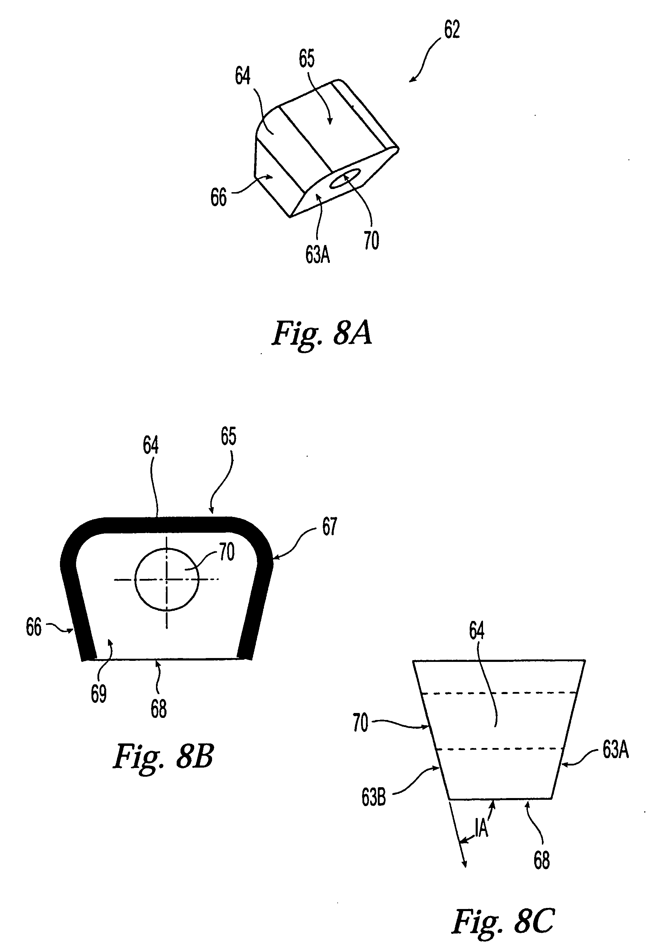

FIGS. 8A, 8B and 8C show a further embodiment of an implant for use in a bilateral laminoplasty procedure. Implant 62 has a first and second ends 63A, 63B, an inner side region 68, an outer side region 65, and sides 66 and 67. The implant 62, like the implant of FIG. 5, has a generally trapezoidal shape when viewed from the side (FIG. 8C). Again, this trapezoidal configuration allows the implant first and second ends 63A, 63B to conform to the cut, angled surfaces of the spinous process segments to which the implant will eventually fuse. As such, inner side region 68 forms angle "IA" with respect to the first and second ends 63A, 63B. In this embodiment, the implant 62 is an allograft, comprising "tri-cortical" bone taken from the crest of the ilium region of the pelvis. Harvesting bone from this segment of the pelvis provides an implant which naturally comprises a thin region 64 of cortical bone on outer side 65, and sides 66 & 67. The inner side region 68 of the implant, as well as the implant body portion 69 comprise cancellous bone. This combination of bone types allows the surgeon to exploit both the good strength characteristics of cortical bone, and the good osteogenic characteristics of cancellous bone in a single implant. In a further embodiment, the implant 62 comprises a cavity 70 which communicates with implant first and second ends 63A & 63B, and which may be used for packing osteogenic material to promote fusion between the implant and the cut spinous process segments.

In a preferred embodiment of the implant 62 of FIG. 8, the implant first and second ends 63A, 63B comprise surface projections to improve pre-fusion retention of the implant 62 between respective cut spinous process segments. Saw-tooth serrations, similar to those illustrated and described with regard to the implant of FIG. 5, may be provided. Again, other suitable surface profiles (e.g. pyramidal teeth, etc.) may also be provided to ensure implant retention.

In a further embodiment of the implant 62 comprising allograft material, first and second ends 63A, 63B may comprise regions of partially, substantially, or fully demineralized cortical bone to further facilitate fusion of the implant to the lamina. Preferably the demineralized bone portion may comprise the entire surface of each first and second ends 63A, 63B of the implant 62. Preferably, the depth of the demineralized portion of will be up to about 2 mm.

In another embodiment, the implant 62 may incorporate a plurality of sutures holes (not shown) similar to those shown in FIG. 5C, to allow the surgeon to secure the implant to the cut spinous process segments. These suture holes may vary in number, size and position, with the only limitation being that their number, size and position should not compromise the strength and integrity of the implant.

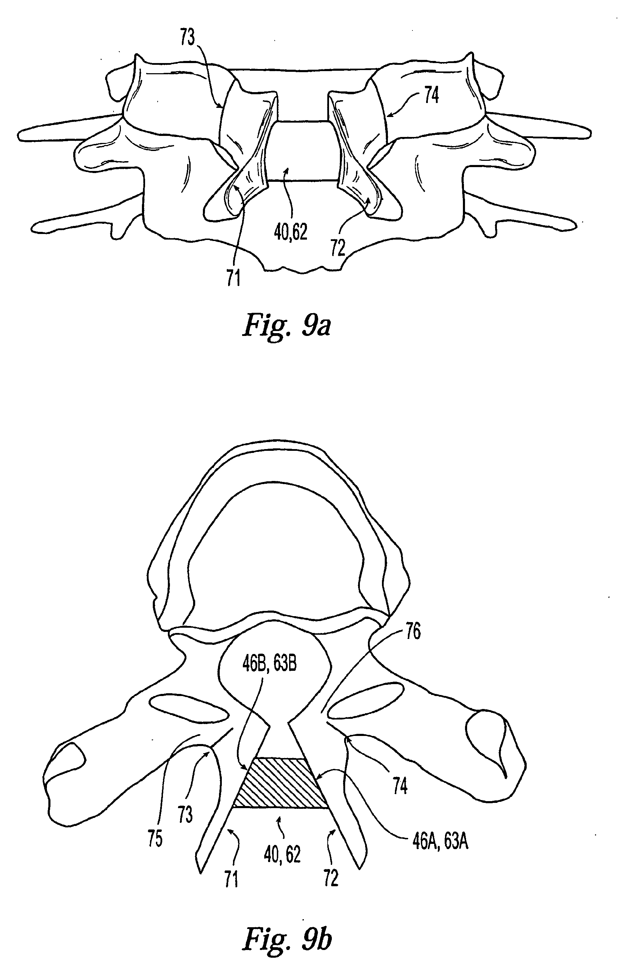

FIGS. 9A and 9B are front and top views of either trapezoidal implants 40, 62 of FIGS. 5, 8 installed in a patient. First and second ends 46A, 46B, 63A, 63B of implant 40, 62 contact cut spinous process segments 72 and 71 respectively. Hinge cuts 73 and 74 in lamina 75, 76 enable the spinous process segments to be "swung out" by the surgeon to facilitate insertion of the implant 40, 62 therebetween.

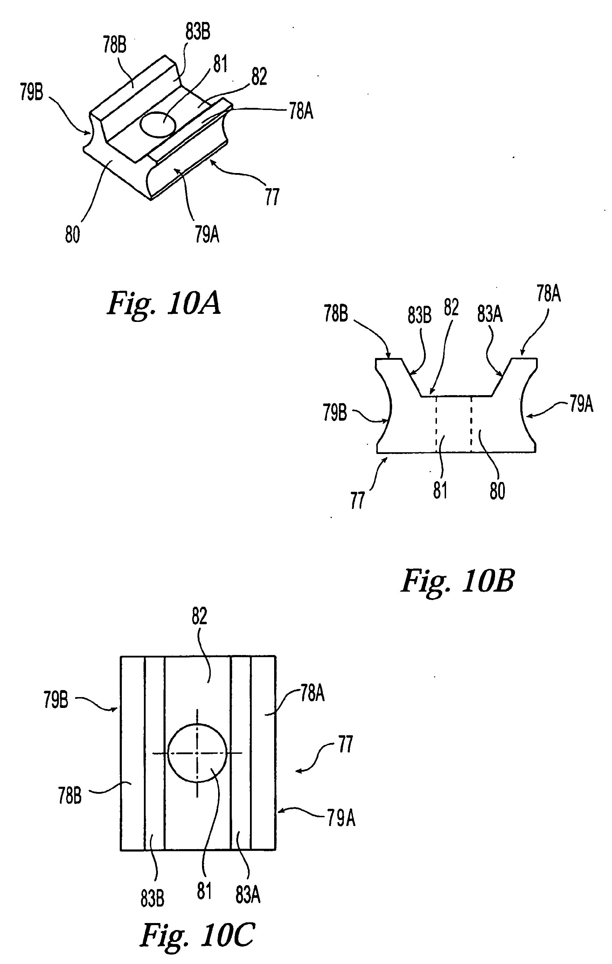

FIGS. 10A, 10B and 10C show a further embodiment of an implant adapted for use in a unilateral laminoplasty procedure. Implant 77 comprises first and second plate portions 78A, 78B for connecting to the opposing segments of cut lamina produced during a unilateral laminoplasty procedure. First and second plate portions 78A, 78B are connected by an intermediate portion 80. The plate portions further comprise respective first and second bone engaging portions 79A, 79B which are configured to engage the opposing cut lamina segments. In a preferred embodiment, first and second bone engaging portions 79A, 79B comprise arcuate surfaces for engaging and cradling the respective cut lamina ends. Arcuate surfaces are particularly suited for this purpose because their concave shape can engage and retain lamina segments residing along different axes, a phenomenon which occurs during the unilateral laminoplasty procedure when a single lamina is cut and the resulting segments are swung out to enlarge the area available for the spinal cord. The swinging out process results in an angle being formed between the segments, and it is this misalignment which the arcuate surfaces of the bone engaging portions 79A & 79B accommodate.

In a further embodiment, the thickness of the intermediate portion 80 may be smaller than the height of the first and second plate portions 78A, 78B.

Implant 77 may be fabricated from any biocompatable metal (e.g. titanium, stainless steel, etc.) or polymer, or the implant may be formed of allograft material. If allograft is used, the implant 77 preferably should be fabricated from cortical bone.

In a further embodiment of the implant 77 comprising allograft material, first and second bone engaging portions 79A, 79B may comprise regions of partially, substantially, or fully demineralized cortical bone to further facilitate fusion of the implant to the lamina segments. Preferably the demineralized bone portion may comprise the entire surface of each first and second bone engaging portions 79A, 79B. Preferably, the depth of the demineralized portion will be up to about 2 mm.

In another embodiment, the implant 77 may incorporate suture hole 80 to allow the surgeon to secure the implant to the cut spinous process segments. Additional suture holes (not shown) may be provided, and may vary in number, size and position, with the only limitation being that their size, position and number preferably should not compromise the strength and integrity of the implant 77.

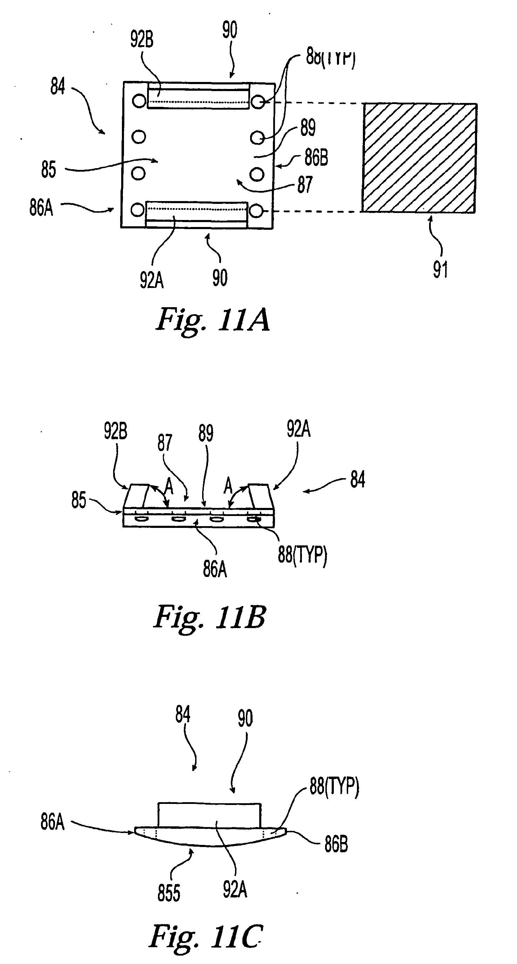

FIGS. 11A, 11B and 11C show a further embodiment of an implant adapted for use in a unilateral laminoplasty procedure. Implant 84 comprises a plate portion 85 having bone engaging portions 86A, 86B, a graft engaging portion 87, and an allograft 91. Bone engaging portions 86A, 86B further comprise a plurality of suture holes 88 configured to allow the surgeon to secure the cut lamina segments to bone engaging portions 86A, 86B Graft engaging portion 87 comprises a graft seating surface 89 and a graft retaining portion 90 configured to retain a correspondingly shaped allograft 91 for engaging the opposing cut lamina segment. In a preferred embodiment, graft retaining portion 90 comprises two raised tabs 92A, 92B, each residing along at least a portion of opposing ends of graft seating surface 89. In a preferred embodiment, raised tabs 92A, 92B are angled slightly toward the center of graft seating surface 89 so as to facilitate retention of allograft 91. Preferably the angle "A" between raised tabs 92A, 92B and graft seating surface 89 will be from about 70 to about 80 degrees; more preferably this angle will be about 75 degrees. Plate portion 85 further comprises a bottom surface 855. When installed, graft 91 comprises the inner side surface of the implant (i.e. the surface which is closest to the spinal canal), while plate bottom surface 855 comprises the outer side surface of the implant (i.e. the surface which faces away from the spinal canal). In a preferred embodiment, bottom surface 855 comprises a convex shape which assumes the rounded contour of the lamina segments. Preferably, this convex surface has a radius of about 18 mm.

Plate portion 85 may be fabricated from any biocompatable metal (e.g. titanium, stainless steel, etc.) or polymer, or it may be made of allograft material. If allograft is used, the plate portion 85 may be fabricated from cortical bone. Graft 91 preferably may be comprised of a cancellous type bone material to facilitate fusion of the implant to the patient's lamina.

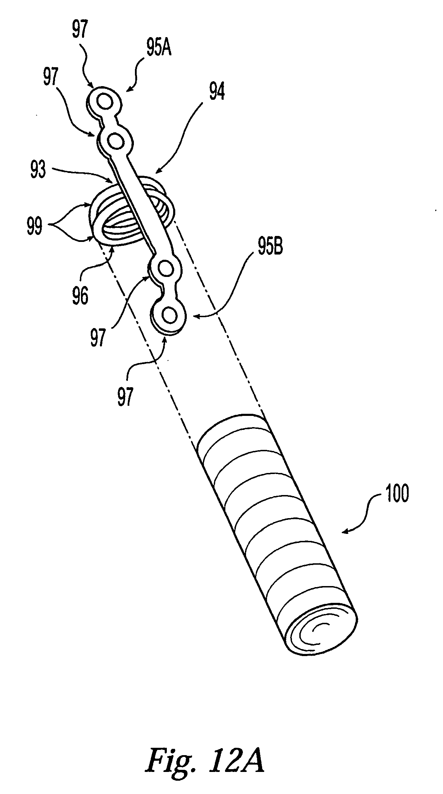

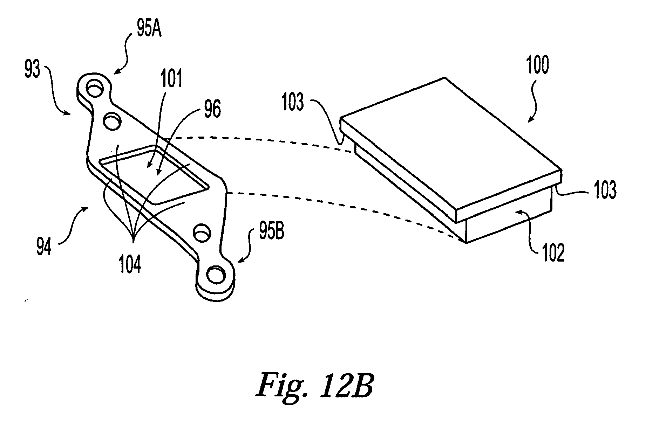

FIGS. 12A and 12B show implant embodiments comprising plates configured to attach directly to the opposing cut segments of lamina produced during a unilateral laminoplasty. These plates are further configured to capture segments of allograft and to engage these segments with the opposing cut segments of lamina to facilitate fusion between the implant and the patient's bone. Plate 93 comprises a body portion 94 having a longitudinal axis and first and second ends 95A, 95B, and a graft retaining portion 96, midway between the ends 95A, 95B, preferably approximately midway between ends 95A, 95B. First and second ends 95A, 95B each comprise a bone engaging portion 97. In a preferred embodiment the bone engaging portion at each first and second end comprises at least one hole suitable for receiving a bone screw 98 (not shown). The bone screws are then used to secure the plate 93 to each opposing segment of lamina. In a further embodiment the bone engaging portions may be hooks capable of grasping bone screws that are installed in the lamina segments.

In the embodiment shown in FIG. 12A, the graft retaining portion 96 comprises a plurality of deformable fingers 99 which are initially arrayed flat along an axis perpendicular to the longitudinal axis of the plate 93. These fingers 99 are capable of being deformed to cradle an allograft 100, preferably cylindrical in shape. Allograft 100 preferably has a length sufficient to engage the cut ends of lamina upon installation, and a diameter of size sufficient to be captured by the deformed fingers 99 of the plate 93.

In the embodiment of FIG. 12B, plate 93 has a graft retaining portion 96 which comprises a hollow region 101, preferably rectangular in shape. A correspondingly configured allograft of cancellous bone is provided having a body 102 capable of being received within the hollow region 101, and further having shoulders 103 which extends beyond the hollow region to contact seating surface 104. In a preferred embodiment, shoulders 103 of allograft 100 are secured to plate 93 using a bone screw 98 placed through bone engaging portion 97.

In a preferred embodiment the plate 93 may be flexible to allow the surgeon to form the body 94 to the individual contour of the patient's spine, thereby achieving a tight fit between components. The plate 93 may be fabricated from a biocompatable metal or other material known in the art that would be suitable for long term retention of an implant and graft.

Following a method not forming part of the invention is described of using an allograft implant according to any of the embodiments shown in FIGS. 1A, 5A, 8A, 10A or 11A which further has partially, substantially, or fully demineralized end segments to promote fusion between opposing segments of lamina or spinous process produced during a unilateral or bilateral laminoplasty procedure. This method comprises the steps of cutting a targeted lamina or spinous process as required for either a unilateral or bilateral laminoplasty procedure, separating the resulting segments of bone a sufficient distance to allow for insertion of an appropriately sized allograft implant, providing an allograft implant having bone engaging surfaces which comprise partially, substantially, or fully demineralized cortical bone to a depth of up to about 2 mm, and contacting the allograft implant bone engaging surfaces with respective cut segments of lamina or spinous process. This method may be augmented, in the case of a unilateral laminoplasty, to include the additional step of installing a plate over the allograft implant to further assist retention of the implant between the bone segments. Where such a plate is provided, the additional steps of providing bone screws or other fasteners to attach the plate to the opposing segments of bone and/or to attach the plate to the implant, may further be included.

A further embodiment of the above method comprises providing an allograft implant according to the above method, which implant further has partially, substantially, or fully demineralized bone flaps capable of receiving bone screws. Providing such an implant allows the surgeon to affirmatively secure the implant to the cut lamina segments, preferably without the need for a separate plate.

A further method not forming part of the invention installing a tri-cortical allograft implant as part of a bilateral laminoplasty procedure comprises the steps of bisecting a targeted spinous process, providing hinge cuts on both adjacent lamina sufficient to allow the spinous process segments to be spread apart, separating the spinous process segments to allow for insertion of an appropriately sized allograft implant, providing an allograft implant having first and second angled bone engaging surfaces which approximate the angle between the bisected and spread spinous process segment cut surfaces, the allograft implant comprising cancellous bone material having a thin outer layer of cortical bone surrounding the cancellous bone, and which cortical bone layer is in communication with the first and second engaging surfaces so as to support the compressive stresses imparted by the cut spinous process segments.

A further method not forming part the invention of using only a screwed plate to maintain the distance between bone ends produced during a unilateral or bilateral laminoplasty procedure is described. This method comprises the steps of cutting a targeted lamina or spinous process as required for the respective laminoplasty procedure, separating the cut bone segments to increase the space available for the spinal canal and associated nerves, providing an appropriately sized plate having first and second ends, wherein each end is configured to allow engagement with the surface of the lamina opposite the surface of the spinal canal and adjacent the cut bone end, and securing first and second ends of the plate to the adjacent bone segments.

In a preferred embodiment of the above method, each first and second end of the plate will have at least one recess suitable for receiving a bone screw, wherein the plate is secured to the adjacent cut bone ends using bone screws. In a further embodiment, two plates may be provided to attach to the adjacent cut bone ends.

## Claims

1. An implant (1) for use in the spinal column, said implant comprising:
A) a body portion having a length, a width and a depth, and configured to be insertable between first and second cut bone segments (10), the body portion having an outer surface (3) and an inner surface (4) defining a substantially hollow portion (7), the body portion further having first (6A) and second ends (6B) which communicate with said hollow portion (7) and a longitudinal axis C_{L}; and wherein
B) the first and second ends (6A,6B) comprise bone engaging portions; wherein at least one of the bone engaging portions comprises a cutout (8A;8B) configured and adapted to engage and retain at least one of the first and second cut bone segments (10).
**characterized in that**
C) the at least one cutout (8A;8B) has a substantially concave arcuate shape; and
D) the at least one cutout (8A;8B) further comprises a centerline C running parallel to the implant longitudinal axis C_{L} dividing said first and second ends (6A,6B), and
E) wherein the centerline C of the at least one cutout (8A;8B) is offset from the longitudinal axis C_{L}.

2. The implant of Claim 1 wherein the perimeter of the outer surface of the implant is a substantially geometric shape.

3. The implant of Claim 2 wherein the geometric shape is an ellipse having a width and a depth.

4. The implant of any of the preceding Claims 1-3 wherein the length ranges from about 11.5 to about 15.5 millimeters, the width ranges from about 8.0 to about 9.0 millimeters and the depth ranges from about 5.5 to about 6.5 millimeters.

5. The implant of Claim 2 wherein the geometric shape is a circle.

6. The implant of any one of the preceding Claims 1-5 wherein the implant comprises a substantially tubular shape.

7. The implant of any of the preceding Claims 1-6 wherein at least a portion of the implant is formed of bone allograft material.

8. The implant of Claim 7 wherein at least a portion of at least one said bone engaging portion is comprised of demineralized cortical bone.

9. The implant of any preceding claims 1-8 further comprising first and second bone flaps, the flaps connecting to and extending from the body portion at the first and second ends, each said flap further comprising at least one hole suitable for receiving a bone fastener for securing said implant to said first and second bone segments.

10. The implant of Claim 9 wherein at least one of the first and second bone flaps is flexible.

11. The implant of either Claims 9 or 10 wherein at least one of the first and second bone flaps is comprised of demineralized cortical bone.

12. The implant of any one of Claims 9-11 wherein at least one of the first and second bone flaps comprises a notch in the region where the at least one bone flap connects to the body portion.

13. The implant of any one of Claims 7-12 wherein said inner surface is defined by the intermedullary canal of the donor bone.

14. The implant of any one of Claims 7-12 wherein said inner surface is configured such that the volume of said substantially hollow portion is greater than the intermedullary canal of the donor bone.

15. The implant of any one of the preceding Claims 1 - 14 wherein both bone engaging portions comprise cutouts and further wherein both cutouts have a substantially concave arcuate shape.

16. The implant of any one of the preceding Claims 1 - 15 wherein the at least one cutout comprises at least two angled faces.

17. The implant of any one of the preceding Claims 1 - 16 further comprising at least one surface defining a hole in communication with said outer surface and said inner surface, suitable for attaching a suture to secure said implant to at least one of said first and second bone segments.

18. The implant of any one of the preceding Claims 1 - 17 wherein at least a portion of the implant is fabricated of biocompatible metal.

19. The implant of any one of the preceding Claims 1 - 17 wherein at least a portion of the implant is fabricated of biocompatible polymer.

20. The implant of any one of the preceding Claims 1 - 19 wherein at least one of the bone engaging portions comprises surface projections configured to retain said implant within said first and second bone segments.

21. The implant of claim 1, wherein said body portion has a longitudinal axis and said first and second bone engaging portions comprise concave cutouts configured and adapted to engage and retain said first and second bone segments, the cutouts further each comprising a centerline running parallel to the implant longitudinal axis and dividing each the cutouts, wherein the centerline of the cutout of the first end is offset from the implant longitudinal axis in one direction, and the centerline of the cutout of the second end is offset from the implant longitudinal axis in the opposite direction.

22. The implant of claim 21, wherein at least one cutout further comprises at least two angled faces.

23. The implant of any one of proceeding claims 21 to 22, wherein the cutouts have a substantially concave arcuate shape.

24. The implant of any one of preceding claims 21 to 23, wherein at least one of the bone engaging portions comprises surface projections configured to retain said implant within said first and second bone segments.

25. The implant of any of the preceding claims 21 to 24, wherein at least a portion of the implant is formed of bone allograft material.

26. The implant of claim 1, wherein said body portion has an inner side region comprising an inner side length and configured to be insertable between said first and second cut bone segments, and wherein at least one of the first and second bone engaging portions is comprised of demineralized allograft material.

27. The implant of Claim 26 wherein the body portion further comprises a wall having an outer surface, and an inner surface defining a substantially hollow portion, wherein the hollow portion is in communication with the first and second ends.

28. The implant of Claim 27 wherein said hollow portion is defined by the intermedullary canal of the donor bone.

29. The implant of any one of Claims 26 to 27 wherein said inner surface is configured such that the volume of said substantially hollow portion is greater than the intermedullary canal of the donor bone.

30. The implant of any one of the preceding Claims 26 to 29 wherein the at least one of said first and second bone engaging portions further comprises surface projections configured to retain said implant within said first and second bone segments.

31. The implant of Claim 30 wherein the surface projections comprise saw-tooth ridges.

32. The implant of Claim 30 wherein the surface projections comprise individual pyramidal teeth.

## Patentansprüche

1. Implantat (1) zur Verwendung in der Wirbelsäule, wobei das Implantat umfasst:
A) einen Körperabschnitt mit einer Länge, einer Breite und einer Tiefe, der konfiguriert ist, um zwischen ersten und zweiten geschnittenen Knochensegmenten (10) einsetzbar zu sein, wobei der Körperabschnitt eine Außenfläche (3) und eine Innenfläche (4) aufweist, die einen im Wesentlichen hohlen Abschnitt (7) definieren, wobei der Körperabschnitt ferner erste Enden (6A) und zweite Enden (6B), die mit dem hohlen Abschnitt (7) in Verbindung stehen, und eine Längsachse C_{L} aufweist; und wobei
B) die ersten und zweiten Enden (6A, 6B) Knocheneingriffsabschnitte umfassen; wobei zumindest einer der Knocheneingriffsabschnitte einen Ausschnitt (8A; 8B) umfasst, der eingerichtet und ausgelegt ist, um mit zumindest einem der ersten und zweiten geschnittenen Knochensegmente (10) in Eingriff zu gelangen und diesen zu halten,
**dadurch gekennzeichnet, dass**
C) der zumindest eine Ausschnitt (8A; 8B) eine im Wesentlichen konkave bogenförmige Form aufweist; und
D) der zumindest eine Ausschnitt (8A; 8B) ferner eine Mittellinie C umfasst, die parallel zur Implantatlängsachse C_{L} verläuft, die die ersten und zweiten Enden (6A, 6B) teilt, und
E) wobei die Mittellinie C des zumindest einen Ausschnitts (8A; 8B) in Bezug auf die Längsachse C_{L} versetzt ist.

2. Implantat nach Anspruch 1, **dadurch gekennzeichnet, dass** der Umfang der Außenfläche des Implantats eine im Wesentlichen geometrische Form ist.

3. Implantat nach Anspruch 2, **dadurch gekennzeichnet, dass** die geometrische Form eine Ellipse mit einer Breite und einer Tiefe ist.

4. Implantat nach einem der vorstehenden Ansprüche 1 bis 3, **dadurch gekennzeichnet, dass** die Länge im Bereich von ungefähr 11,5 bis ungefähr 15,5 mm liegt, **dadurch gekennzeichnet, dass** die Breite im Bereich von ungefähr 8,0 bis ungefähr 9,0 mm liegt und wobei die Tiefe im Bereich von ungefähr 5,5 bis ungefähr 6,5 mm liegt.

5. Implantat nach Anspruch 2, **dadurch gekennzeichnet, dass** die geometrische Form ein Kreis ist.

6. Implantat nach einem der vorstehenden Ansprüche 1 bis 5, **dadurch gekennzeichnet, dass** das Implantat eine im Wesentlichen rohrförmige Form umfasst.

7. Implantat nach einem der vorstehenden Ansprüche 1 bis 6, **dadurch gekennzeichnet, dass** zumindest ein Teil des Implantats aus Knochenallotransplantatmaterial gebildet ist.

8. Implantat nach Anspruch 7, **dadurch gekennzeichnet, dass** zumindest ein Teil des zumindest einen Knocheneingriffsabschnitts entmineralisierten kortikalen Knochen enthält.

9. Implantat nach einem der vorstehenden Ansprüche 1 bis 8, ferner umfassend erste und zweite Knochendeckel, wobei sich die Deckel mit dem Körperabschnitt an den ersten und zweiten Enden verbinden und sich von diesem erstrecken, wobei jeder Deckel darüber hinaus zumindest ein Loch umfasst, das geeignet ist, um einen Knochenbefestiger aufzunehmen, um das Implantat an den ersten und zweiten Knochensegmenten zu befestigen.

10. Implantat nach Anspruch 9, **dadurch gekennzeichnet, dass** zumindest einer der ersten und zweiten Knochendeckel flexibel ist.

11. Implantat nach Anspruch 9 oder 10, **dadurch gekennzeichnet, dass** zumindest einer der ersten und zweiten Knochendeckel entmineralisierten kortikalen Knochen enthält.

12. Implantat nach einem der Ansprüche 9 bis 11, **dadurch gekennzeichnet, dass** zumindest einer der ersten und zweiten Knochendeckel eine Kerbe in dem Bereich umfasst, in dem sich der zumindest eine Knochendeckel mit dem Körperabschnitt verbindet.

13. Implantat nach einem der Ansprüche 7 bis 12, **dadurch gekennzeichnet, dass** die Innenfläche vom intermedullären Kanal des Spenderknochens definiert ist.

14. Implantat nach einem der Ansprüche 7 bis 12, **dadurch gekennzeichnet, dass** die Innenfläche derart eingerichtet ist, dass das Volumen des im Wesentlichen hohlen Abschnitts größer als der intermedulläre Kanal des Spenderknochens ist.

15. Implantat nach einem der vorstehenden Ansprüche 1 bis 14, **dadurch gekennzeichnet, dass** beide Knocheneingriffsabschnitte Ausschnitte umfassen, und ferner wobei beide Ausschnitte eine im Wesentlichen konkave bogenförmige Form aufweisen.

16. Implantat nach einem der vorstehenden Ansprüche 1 bis 15, **dadurch gekennzeichnet, dass** der zumindest eine Ausschnitt zumindest zwei abgewinkelte Flächen umfasst.

17. Implantat nach einem der vorstehenden Ansprüche 1 bis 16, ferner umfassend zumindest eine Fläche, die ein Loch definiert, das mit der Außenfläche und der Innenfläche in Verbindung steht, geeignet, um eine Naht anzuhaften, um das Implantat an zumindest einem der ersten und zweiten Knochensegmente zu befestigen.

18. Implantat nach einem der vorstehenden Ansprüche 1 bis 17, **dadurch gekennzeichnet, dass** zumindest ein Teil des Implantats aus biokompatiblem Metall hergestellt ist.

19. Implantat nach einem der vorstehenden Ansprüche 1 bis 17, **dadurch gekennzeichnet, dass** zumindest ein Teil des Implantats aus biokompatiblem Polymer hergestellt ist.

20. Implantat nach einem der vorstehenden Ansprüche 1 bis 19, **dadurch gekennzeichnet, dass** zumindest einer der Knocheneingriffsabschnitte Flächenvorsprünge umfasst, die eingerichtet sind, um das Implantat innerhalb der ersten und zweiten Knochensegmente zu halten.

21. Implantat nach Anspruch 1, **dadurch gekennzeichnet, dass** der Körperabschnitt eine Längsachse aufweist, und wobei die ersten und zweiten Knocheneingriffsabschnitte konkave Ausschnitte umfassen, die eingerichtet und ausgelegt sind, um mit den ersten und zweiten Knochensegmenten in Eingriff zu gelangen und diese zu halten, wobei die Ausschnitte ferner jeweils eine Mittellinie umfassen, die parallel zur Implantatlängsachse verläuft und die Ausschnitte jeweils teilt, wobei die Mittellinie des Ausschnitts des ersten Endes in Bezug auf die Implantatlängsachse in einer Richtung versetzt ist, und wobei die Mittellinie des Ausschnitts des zweiten Endes in Bezug auf die Implantatlängsachse in der entgegengesetzten Richtung versetzt ist.

22. Implantat nach Anspruch 21, **dadurch gekennzeichnet, dass** zumindest ein Ausschnitt ferner zumindest zwei abgewinkelte Flächen umfasst.

23. Implantat nach einem der vorstehenden Ansprüche 21 bis 22, **dadurch gekennzeichnet, dass** die Ausschnitte eine im Wesentlichen konkave bogenförmige Form aufweisen.

24. Implantat nach einem der vorstehenden Ansprüche 21 bis 23, **dadurch gekennzeichnet, dass** zumindest einer der Knocheneingriffsabschnitte Flächenvorsprünge umfasst, die eingerichtet sind, um das Implantat innerhalb der ersten und zweiten Knochensegmente zu halten.

25. Implantat nach einem der vorstehenden Ansprüche 21 bis 24, **dadurch gekennzeichnet, dass** zumindest ein Teil des Implantats aus Knochenallotransplantatmaterial gebildet ist.

26. Implantat nach Anspruch 1, **dadurch gekennzeichnet, dass** der Körperabschnitt einen Innenseitenbereich aufweist, der eine Innenseitenlänge umfasst und eingerichtet ist, um zwischen den ersten und zweiten geschnittenen Knochensegmenten einsetzbar zu sein, und wobei zumindest einer der ersten und zweiten Knocheneingriffsabschnitte aus entmineralisiertem Allotransplantatmaterial besteht.

27. Implantat nach Anspruch 26, **dadurch gekennzeichnet, dass** der Körperabschnitt ferner eine Wand mit einer Außenfläche und einer Innenfläche umfasst, die einen im Wesentlichen hohlen Abschnitt definieren, wobei der hohle Abschnitt mit den ersten und zweiten Enden in Verbindung steht.

28. Implantat nach Anspruch 27, **dadurch gekennzeichnet, dass** der hohle Abschnitt vom intermedullären Kanal des Spenderknochens definiert ist.

29. Implantat nach einem der Ansprüche 26 bis 27, **dadurch gekennzeichnet, dass** die Innenfläche derart eingerichtet ist, dass das Volumen des im Wesentlichen hohlen Abschnitts größer als der intermedulläre Kanal des Spenderknochens ist.

30. Implantat nach einem der vorstehenden Ansprüche 26 bis 29, **dadurch gekennzeichnet, dass** der zumindest eine der ersten und zweiten Knocheneingriffsabschnitte ferner Flächenvorsprünge umfasst, die eingerichtet sind, um das Implantat innerhalb der ersten und zweiten Knochensegmente zu erhalten.

31. Implantat nach Anspruch 30, **dadurch gekennzeichnet, dass** die Flächenvorsprünge Sägezahnerhöhungen umfassen.

32. Implantat nach Anspruch 30, **dadurch gekennzeichnet, dass** die Flächenvorsprünge einzelne pyramidenförmige Zähne umfassen.

## Revendications

1. Implant (1) à utiliser dans la colonne vertébrale, ledit implant comprenant :
A) une portion de corps ayant une longueur, une largeur et une profondeur, et configurée pour être insérable entre les premier et second segments osseux coupés (10), la portion de corps ayant une surface externe (3) et une surface interne (4) définissant une portion sensiblement creuse (7), la portion de corps ayant en outre des première (6A) et seconde (6B) extrémités qui communiquent avec ladite portion creuse (7) et un axe longitudinal C_{L} ; et dans lequel
B) les première et seconde extrémités (6A, 6B) comprennent des portions d'engagement d'os ; dans lequel au moins l'une des portions d'engagement d'os comprend une découpe (8A ; 8B) configurée et adaptée pour engager et retenir au moins l'un des premier et second segments osseux coupés (10),
**caractérisé en ce que**
C) la au moins une découpe (8A ; 8B) a une forme sensiblement concave arquée; et
D) la au moins une découpe (8A ; 8B) comprend en outre une ligne médiane C courant parallèlement à l'axe longitudinal C_{L} de l'implant divisant lesdites première et seconde extrémités (6A, 6B), et
E) dans lequel la ligne médiane C de la au moins une découpe (8A ; 8B) est décalée de l'axe longitudinal C_{L}.

2. Implant selon la revendication 1, dans lequel le périmètre de la surface externe de l'implant est une forme sensiblement géométrique.

3. Implant selon la revendication 2, dans lequel la forme géométrique est une ellipse ayant une largeur et une profondeur.

4. Implant selon l'une quelconque des revendications 1 à 3, dans lequel la longueur varie d'environ 11,5 à environ 15,5 millimètres, la largeur varie d'environ 8,0 à environ 9,0 millimètres et la profondeur varie d'environ 5,5 à environ 6,5 millimètres.

5. Implant selon la revendication 2, dans lequel la forme géométrique est un cercle.

6. Implant selon l'une quelconque des revendications 1 à 5 précédentes, dans lequel l'implant comprend une forme sensiblement tubulaire.

7. Implant selon l'une quelconque des revendications 1 à 6 précédentes, dans lequel au moins une portion de l'implant est formée d'un matériau d'allogreffe osseuse.

8. Implant selon la revendication 7, dans lequel au moins une portion de ladite au moins une portion d'engagement d'os est composée d'os cortical déminéralisé.

9. Implant selon l'une quelconque des revendications 1 à 8 précédentes, comprenant en outre un ou plusieurs volets osseux, les volets se raccordant à et s'étendant depuis la portion de corps au niveau des première et seconde extrémités, chaque dit volet comprenant en outre au moins un trou approprié pour recevoir un moyen de fixation osseuse permettant d'arrimer ledit implant auxdits premier et second segments osseux.

10. Implant selon la revendication 9, dans lequel au moins l'un des premier et second volets osseux est flexible.

11. Implant selon l'une quelconque des revendications 9 ou 10, dans lequel au moins l'un des premier et second volets osseux est composé d'os cortical déminéralisé.

12. Implant selon l'une quelconque des revendications 9 à 11, dans lequel au moins l'un des premier et second volets osseux comprend une encoche dans la région où le au moins un volet osseux se raccorde à la portion de corps.

13. Implant selon l'une quelconque des revendications 7 à 12, dans lequel ladite surface interne est définie par le canal intermédullaire de l'os donneur.

14. Implant selon l'une quelconque des revendications 7 à 12, dans lequel ladite surface interne est configurée de telle sorte que le volume de ladite portion sensiblement creuse est plus grand que le canal intermédullaire de l'os donneur.

15. Implant selon l'une quelconque des revendications 1 à 14 précédentes, dans lequel les deux portions d'engagement d'os comprennent des découpes et dans lequel en outre, les deux découpes ont une forme arquée sensiblement concave.

16. Implant selon l'une quelconque des revendications 1 à 15 précédentes, dans lequel la au moins une découpe comprend au moins deux faces obliques.

17. Implant selon l'une quelconque des revendications 1 à 16, comprenant en outre au moins une surface définissant un trou en communication avec ladite surface externe et ladite surface interne, appropriée pour attacher une suture en vue d'arrimer ledit implant à au moins l'un des premier et second segments osseux.

18. Implant selon l'une quelconque des revendications 1 à 17 précédentes, dans lequel au moins une portion de l'implant est fabriquée en métal biocompatible.

19. Implant selon l'une quelconque des revendications 1 à 17 précédentes, dans lequel au moins une portion de l'implant est fabriquée en polymère biocompatible.

20. Implant selon l'une quelconque des revendications 1 à 19 précédentes, dans lequel au moins l'une des portions d'engagement d'os comprend des saillies de surface configurées pour retenir ledit implant au sein desdits premier et second segments osseux.

21. Implant selon la revendication 1, dans lequel ladite portion de corps a un axe longitudinal et lesdites première et seconde portions d'engagement d'os comprennent des découpes concaves configurées et adaptées pour engager et retenir lesdits premier et second segments osseux, les découpes comprenant en outre chacune une ligne médiane courant parallèlement à l'axe longitudinal de l'implant et divisant chacune des découpes, où la ligne médiane de la découpe de la première extrémité est décalée de l'axe longitudinal de l'implant dans une direction, et la ligne médiane de découpe de la seconde extrémité est décalée de l'axe longitudinal de l'implant dans la direction opposée.

22. Implant selon la revendication 21, dans lequel au moins une découpe comprend en outre au moins deux faces obliques.

23. Implant selon l'une quelconque des revendications 21 et 22 précédentes, dans lequel les découpes ont une forme arquée sensiblement concave.

24. Implant selon l'une quelconque des revendications 21 à 23 précédentes, dans lequel au moins l'une des portions d'engagement d'os comprend des saillies de surface configurées pour retenir ledit implant au sein desdits premier et second segments osseux.

25. Implant selon l'une quelconque des revendications 21 à 24 précédentes, dans lequel au moins une portion de l'implant est formée d'un matériau d'allogreffe osseuse.

26. Implant selon la revendication 1, dans lequel ladite portion de corps a une région latérale interne comprenant une longueur latérale interne et configurée pour être insérable entre lesdits premier et second segments osseux coupés, et dans lequel au moins une des première et seconde portions d'engagement d'os est composée de matériau d'allogreffe déminéralisée.

27. Implant selon la revendication 26, dans lequel la portion de corps comprend en outre une paroi ayant une surface externe, et une surface interne définissant une portion sensiblement creuse, dans lequel la portion creuse est en communication avec les première et seconde extrémités.

28. Implant selon la revendication 27, dans lequel ladite portion creuse est définie par le canal intermédullaire de l'os donneur.

29. Implant selon l'une quelconque des revendications 26 à 27, dans lequel la surface interne est configurée de telle sorte que le volume de ladite portion sensiblement creuse est plus grand que le canal intermédullaire de l'os donneur.

30. Implant selon l'une quelconque des revendications 26 à 29 précédentes, dans lequel au moins l'une desdites première et seconde portions d'engagement d'os comprend en outre des saillies de surface configurées pour retenir ledit implant au sein desdits premier et second segments osseux.

31. Implant selon la revendication 30, dans lequel les saillies de surface comprennent des arêtes en dents de scie.

32. Implant selon la revendication 30, dans lequel les saillies de surface comprennent des dents pyramidales individuelles.
